Europäisches Patentamt

European Patent Office

Office européen des brevets

(11) Veröffentlichungsnummer: **0 188 004**

**A2**

(12) ## EUROPÄISCHE PATENTANMELDUNG

(21) Anmeldenummer: **85116636.3**

(22) Anmeldetag: **28.12.85**

(51) Int. Cl.⁴: **A 23 K 1/165**

(30) Priorität: **12.01.85 DE 3500912**

(43) Veröffentlichungstag der Anmeldung:
**23.07.86 Patentblatt 86/30**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Gropp, Jürgen, Prof. Dr.**
**Bahnhofstrasse 73**
**D-8031 Eichenau(DE)**

(72) Erfinder: **Schlingmann, Merten, Dr.**
**Schneidhainer Strasse 32a**
**D-6240 Königstein/Taunus(DD)**

(72) Erfinder: **Präve, Paul, Prof. Dr.**
**Kronberger Weg 7**
**D-6231 Sulzbach(DE)**

(72) Erfinder: **Faust, Uwe, Dr.**
**Kelkheimer Strasse 27**
**D-6233 Kelkheim (Taunus)(DE)**

(54) **Veredelung von rohproteinen.**

(57) Nucleinsäurereiche Rohproteine sind als Tierfutter besser geeignet, wenn die Nucleinsäuren enzymatisch abgebaut werden.

EP 0 188 004 A2

Croydon Printing Company Ltd

## Veredelung von Rohproteinen

Industrielle Fermentationsprozesse zur Herstellung von Biomasse für Futtermittel haben eine lange Tradition (Hefegewinnung aus Sulfitablaugen) und waren in den letzten Jahren Gegenstand neuer Verfahrensentwicklungen (Bakteriengewinnung auf Basis Methanol).

Biomassen aus Fermentationsverfahren für das Anwendungsgebiet der Tierernährung wie Kälber- und Geflügelmast, Fischaufzucht, Legehennenfutter, Schweinemast und Haustierfutter werden auch als Bioproteine bezeichnet und enthalten je nach Art des Mikroorganismus (Hefe, Bakterium), der verwendeten Kohlenstoffquelle (Sulfitablaugen, Ethanol, Methanol, Paraffinfraktionen) sowie Art der Fermentations- und Aufarbeitungstechnologie unterschiedliche Mengen an Proteinen, Lipiden, Kohlenhydraten und Nucleinsäuren [Ribonucleinsäuren (RNS) und Desoxyribonucleinsäuren, (DNS)].

Während der nutritive Wert der Biomasse in erster Linie eine Funktion des Proteingehaltes und der Aminosäurezusammensetzung und -verfügbarkeit ist und die Lipide- und Kohlenhydratfraktion zum Energiegehalt der Gesamtfuttermischung beitragen, ist der Nucleinsäureanteil eine Nebenkomponente, deren Stickstoffanteil zum Aufbau von Körpersubstanz nicht oder in nur geringem Anteil genutzt werden kann.

Abhängig vom tierartspezifischen Stoffwechsel werden die stickstoffhaltigen Bausteine von RNS und DNS (Purine, Pyrimidine) zu Metaboliten abgebaut, die in Form gut wasserlöslicher (Allantoin) oder schlecht löslicher Produkte (Harnsäure) ausgeschieden werden.

- 2 -

Deshalb wird der Nucleinsäuregehalt in Futtermischungen begrenzt, um überhöhte Stoffwechsel- oder Organbelastungen durch Nucleinsäureabbauprodukte zu vermeiden. Ein Zusammenhang zwischen Nucleinsäuregehalt und nutritiver Verfügbarkeit (Verdaulichkeit und Verträglickeit) von Bioproteinen in Abhängigkeit vom Molekulargewicht der Nucleinsäuren (RNS und DNS) wurden bisher dagegen nicht beschrieben.

Es wurde nun gefunden, daß Nucleinsäuren neben den vorstehend genannten Effekten im Stoffwechsel, die sich aus der chemischen Zusammensetzung von RNS bzw. DNS (Ribose, Desoxyribose, Phosphorsäure, Purine, Pyrimidine) ergeben, einen bisher unbekannten negativen Einfluß auf die Verdaulichkeit von Bioproteinen haben, der durch das hohe Molekulargewicht dieser Verbindungen verursacht wird.

Besonders die Polynucleotide der DNS erreichen Molekulargewichtsbereiche von $1 \cdot 10^5$ bis $1 \cdot 10^7$, während die kürzeren Polynucleotidstränge der RNS Molekulargewichte von 5000 — 50 000 haben.

Der Einfluß des Molekulargewichtes von Nucleinsäure auf die Verdaulichkeit und Verträglichkeit von Bioproteinen wurde erstmals in Fütterungsversuchen bei Küken beobachtet und daraus ein Untersuchungsmodell für die Bestimmung des Molekulargewichtseffektes auf die Verdauungsorgane entwickelt.

Der negative Effekt von hochmolekularen Nucleinsäuren in der Futtermischung bewirkt konzentrationsabhängig eine Erhöhung des Darmgewichtes im Verhältnis zum Körpergewicht und dadurch eine allgemeine Verschlechterung des Stoffwechsels durch negative Veränderung der Resorptionsvorgänge. Schlechtere Wachstumsergebnisse und damit eine scheinbar geringere Proteinwertigkeit nucleinsäurehaltiger Bioproteine sind die primär zu beobachtenden Folgen.

Es ist bekannt, daß sich das Molekulargewicht von RNS und DNS durch pH-Wert, Temperatur und Salzgehalt in wäßrigem Milieu verringern läßt. Während RNS im alkalischen Bereich hydrolysiert, kann DNS unter sauren Bedingungen abgebaut werden. Der Hydrolysegrad (Verringerung des Molekulargewichtes) ist dabei abhängig von den gewählten Bedingungen, wobei selbst bei höherer Tempratur (80-90°C) und pH-Werten von 8-11 für RNS bzw. pH 1,5 - 4,5 für DNS und Behandlungszeiten von 1-4 Stunden nur eine partielle Hydrolyse zu Oligonucleotiden mit Molekulargewichten von 500 - 10 000 bei RNS und 1000-40 000 bei DNS erreicht wird. Dabei werden außerdem die anderen Bestandteile des Bioproteins, insbesondere die Proteine, negativ beeinflußt (Farbe, Geschmack, Denaturierung, Salzgehalt, Racemisierung) und dadurch der nutritive Wert herabgesetzt. Außerdem genügt die partielle Hydrolyse von RNS und DNS nicht, um ihre Nachteile auf die Verdaulichkeit zu eliminieren.

Die Nachteile hochmolekularer Polynucleotide auf die Verträglichkeit von Rohbioproteinen wird erfindungsgemäß dadurch behoben, daß RNS und DNS durch Enzyme unter milden Bedingungen weitgehend, vorzugsweise quantitativ, zu oligomeren, vorzugsweise monomeren, Nucleotiden abgebaut werden.

Die Erfindung betrifft deshalb die Verwendung von nucleinsäurereichen Rohproteinen, in denen durch Behandlung mit Nucleasen die hochmolekularen Nucleinsäuren gespalten wurden, zur Tierernährung.

Als Rohbioproteine kommen in erster Linie mikrobielle Proteine, vor allem Einzellerproteine wie Hefen und insbesondere Bakterienproteine, aber auch nucleinsäurereiche andere
Materialien wie Fischmehl oder Innereien in Betracht.

Die verwendeten Enzyme sind Nucleasen, vor allem Phosphodiesterasen, bevorzugt 5'-Phosphodiesterasen, die RNS und
DNS zu den entsprechenden monomeren 3'- bzw. 5'-Nucleotiden hydrolysieren.

Phosphodiesterasen sind in der Natur weit verbreitet und
lassen sich sowohl aus Mikroorganismen (Streptomyceten,
Penicillium, Staphylococcen), tierischen Zellen oder Produkten (Rinderherz, Rinder- und Kalbsmilz, Schlangengiften) oder pflanzlichen Zellen (Weizenkeimlinge) isolieren.

Da Phosphodiesterasen nur die molekulare Struktur von RNS
und DNS hydrolysieren, treten keine unerwünschten Nebenreaktionen an den anderen Bioproteinkomponenten (Lipide,
Kohlenhydrate, Eiweiß) auf.

Phosphodiesterasen können die Zellwand von Mikroorganismen
durchdringen, weshalb eine besondere Vorbehandlung des
Rohbioproteins für die enzymatische Hydrolyse von RNS und
DNS nicht erforderlich ist. Deshalb läßt sich dieser Verfahrensschritt in jeden Aufarbeitungsablauf von fermentativ hergestellter Biomasse integrieren.

Die erforderliche Enzymkonzentration (g) steht zur Substratkonzentration (g) im Verhältnis 1 : 1 000 bis
1 : 500 000, bevorzugt 1 : 10 000 bis 1 : 100 000.

Neben Rohenzympräparationen aus Mikroorganismen und Pflanzen werden käufliche Enzyme wie

0188004

1. Boehringer Mannheim: Phosphodiesterase     EC 3.1.4.1
        aus Crotalus durissus

2.       "             Nuclease P 1
        aus Penicillium      EC 3.1.30.1
        citrinum

3. Sigma:           Nuclease         EC 3.1.30.1
        Mung bean nuclease

4.     "              Nuclease P 1
        aus Penicillium
        citrinum

5. Amano:          Nuclease RP

bevorzugt. Die Enzyme können dabei je nach Verfahrensweise in ihrer ursprünglichen Form als auch in adsorptiv, ionisch oder kovalent auf einen Träger fixierter Form eingesetzt werden.

Das Reaktionsmilieu für die enzymatische Hydrolyse ist Wasser oder Mischungen mit wassermischbaren organischen Lösemitteln, wobei je nach Biomassekonzentration und -art die Nucleinsäurekonzentration bei 0,05 - 10, vorzugsweise 0,3 - 7, besonders bevorzugt 1 - 5,Gew.-% liegt. Der bevorzugte pH-Bereich ist 4,5 - 6,5, besonders pH 5-6, der während der Hydrolysereaktion durch Abpuffern der entstehenden Säuregruppen der oligo- bzw. monomeren Nucleotide mit Laugen wie $NH_4OH$, $NaOH$, $KOH$, $Ca(OH)_2$ oder basischen Salzen im bevorzugten Bereich gehalten wird.

Die Reaktion erfolgt bei einer Temperatur von 30 bis 60°C, bevorzugt 45 - 55°C. Der Zeitbedarf für die Hydrolyse ist abhängig von der Art der eingesetzten Biomasse, der Temperatur und dem Enzym und beträgt im allgemeinen 0,5 - 8 Stunden.

Zur Bestimmung des Hydrolyseverlaufes von RNS und DNS kann die Konzentration der Nucleotide mittels HPLC bestimmt werden:

Stationäre Phae: LiChrosorb$^{(R)}$ RP 18 7 μ (Merck)
Säule l = 20 cm, ⌀ 0,4 cm

Mobile Phase: bidest. $H_2O$ mit konz. $H_3PO_4$ (pH 2,1)
Flow rate: 2,0 ml/min
Druck: ~ 100 bar
Wellenlänge: 254 nm

Referenzsubstanzen
a) für RNS: 5'-Nucleotide (5'-CMP, 5'-UMP, 5'-AMP, 5'-GMP)
b) für DNS: Desoxy-5'-nucleotide (d-5'-CMP, d-5'-AMP, d-5'-GMP, d-5'-TMP)

Tierexperimentelle Untersuchungen

Zur Ermittlung des Verhältnisses von Darmgewicht zu Körpergewicht werden Hühnerküken nach einer Vorperiode von 4 Tagen mit einem vitamin- und spurenelementarmen Futter mit nährstoffäquivalenten Kontroll- und Versuchsrationen über ca. 9 Tage gefüttert. Dabei ist es unerheblich, ob die Versuchsperiode unmittelbar an die Vorperiode anschließt oder eine mehr oder weniger lange Zwischenperiode mit Kontrollfutter (z.B. 1-21 Tage) der Versuchsperiode vorgeschaltet wird.

Die Rationen der eigentlichen Versuchsreihen enthalten ca. 31 % Reinprotein (Aminosäurenprotein), 1,88 % Ca, 1,5 % P, 0,18 % Na und 0,6 % K; sie sind isoenergetisch (auf der Basis der umsetzbaren Energie, ca. 14 MJ/kg) berechnet. Sojaprotein dient in der Kontrollration als einzige Proteinquelle. Es wird mit DL-Methionin und Glycin bedarfsdeckend ergänzt. In der Versuchsration dient Bakterienprotein als einzige Proteinquelle. Es ist bedarfsdeckend mit DL-Methionin und L-Arginin-HCl supplementiert.

Tabelle 1 orientiert beispielhaft über die Rationszusammensetzung.

Tabelle 1

Zusammensetzung (Gew.-%) der Kontroll- und einer Versuchsration

|  | Kontrollration | Versuchsration |
|---|---|---|
| Sojafeinmehl | 29,26 | |
| Sojaproteinsiolat (PURINA RP 100) | 22,36 | |
| Bakterienprotein | - | 48,40 |
| DL-Methionin | 0,45 | 0,45 |
| L-Arginin·HCl | - | 0,20 |
| Glycin | 0,65 | - |
| Vitamin-VM[1] | 1,00 | 1,00 |
| Spurenelement-VM[2] | 0,10 | 0,10 |
| Ca-Propionat | 0,50 | 0,50 |
| Mineralstoffe | 7,55 | 4,80 |
| Maisstärke | 31,13 | 41,05 |
| Sojaöl | 7,00 | 3,50 |
| | 100,00 | 100,00 |

[1] Vitamin-Vormischung, enthält in 10 g (pro kg der Ration) 10 000 I.E. Vitamin A, Vitamin D (Menge jeweils in der Versuchsbeschreibung angegeben), 10 mg Vitamin E, 2 mg Vitamin $K_3$, 4 mg Thiamin, 8 mg

Riboflavin, 2 mg Pyridoxin, 20 µg Vitamin $B_{12}$, 0,1 mg Biotin, 1,5 mg Folsäure, 20 mg Ca-Panthotenat, 60 mg Nicotinsäure, 1 g Cholinchlorid und 100 mg Ascorbinsäure.

2) Spurenelement-Vormischung, enthält in 1 g (= 200 kg der Ration): 100 mg Fe, 80 mg Zn, 80 mg Mn, 12 mg Cu, 2,5 mg J, 1 mg Co, 0,5 mg Se und 5 mg F.

Die Mastküken werden in Käfigen auf Drahtrosten bei 24 h Dauerlicht gehalten. Sie können Futter und Wasser (aus einer Beckentränke) ad libitum aufnehmen. Das Futter wird grundsätzlich pelletiert (∅ 3 mm) verabreicht.

Als Versuchsparameter dienen die Gewichtsentwicklung, das absolute und relative Gewicht des Dünndarmes (ohne Darminhalt) sowie dessen Länge.

<u>Ergebnisse</u>

In einer Serie von verschiedenen Versuchen konnte gezeigt werden, daß Bakterienprotein - auch peroxidbehandelt - zu einer Verringerung der Lebendmassezunahme im Vergleich zur Sojakontrollgruppe sowie zu einer Erhöhung des Dünndarmgewichtes führt. Tabelle 2 orientiert über die Ergebnisse.

Aus dem Vergleich der Versuchsdaten geht hervor, daß Bakterienprotein (roh oder peroxidbehandelt) die Lebendmasse-Zunahme im Versuchszeitraum auf 49 % (± 13 %) des Sojakontrollwertes reduzierte. Die relativen Darmgewichte lagen bei 152 ± 12 % des Kontrollgruppenwertes.

Tabelle 3 zeigt die analogen Werte bei Verwendung nucleasebehandelter Bakterienprotein-Chargen.

Aus Vergleichen mit nucleasebehandeltem Bakterienprotein ergibt sich, daß das relative Darmgewicht mit 128 ± 16 % des Kontrollwertes wesentlich geringer erhöht war als ohne Nucleasebehandlung. Wird zusätzlich noch dem Umstand Rechnung getragen, daß das relative Darmgewicht mit zunehmendem Körpergewicht abfällt, so relativieren sich diese Unterschiede weiter, da die mit Bakterienprotein gefütterten Tiere immer deutlich leichter als die Kontrolltiere waren.

Insofern konnte bioexperimentell belegt werden, daß die Nuclease-Behandlung von Bioprotein ein geeignetes Verfahren ist, um Nebeneffekte, die bei der Verabreichung unüblich hoher Mengen an Bakterienprotein bei Küken auftreten, zu vermindern. Insbesondere wird das Dünndarmgewicht günstig beeinflußt. Die gemessenen Dünndarmlängen, die nach Gabe von Bakterienprotein ohne Nuclease-Behandlung deutlich größer waren als bei den Kontrolltieren, waren bei Nuclease-behandeltem Bakterienprotein den Kontrollwerten angeglichen.

Tabelle 2: Versuchsergebnisse mit unbehandeltem Bakterienprotein

VB = Versuchsbeginn, VE = Versuchsende, LM = Lebendmasse, s = Streuung

| Versuch | I | | | | II | | | | III | | | |
|---|---|---|---|---|---|---|---|---|---|---|---|---|
| Proteinquelle | Soja | Bakterien-protein | Soja | Bakterien-protein | Soja | Bakterien-protein | Soja | Bakterien-protein | Soja | Bakterien-protein | Soja | Bakt.-protein |
| Vit.$D_3$-Menge | 500 IE/kg | | 10.000 IE/kg | | 500 IE/kg | | 200 IE/Tier | | 13 000 IE/kg Futter | | | |
| Tierzahl | 35 | 35 | 34 | 27 | 24 | 21 | 28 | 28 | 33 | 28 | 29 | 32 |
| Vorperiode (Tg) | 4 | 4 | 4 | 4 | 4 | 4 | 4 | 4 | 4 | 4 | 4 | 4 |
| Zwischenp. (Tg) | 0 | 0 | 0 | 0 | 0 | 0 | 0 | 0 | 10 | 10 | 21 | 21 |
| Versuchsp. (Tg) | 7 | 7 | 7 | 7 | 10 | 10 | 10 | 10 | 11 | 11 | 9 | 9 |
| LM (g) bei VB | 78 | 78 | 78 | 79 | 76 | 76 | 76 | 76 | 277 | 268 | 680 | 678 |
| LM (g) bei VE | 254 | 181 | 268 | 204 | 319 | 165 | 333 | 183 | 686 | 425 | 1156 | 865 |
| ± s | 35 | 18 | 22 | 31 | 37 | 19 | 30 | 31 | 109 | 98 | 168 | 215 |
| Relativ | (100) | (71) | (100) | (76) | (100) | (52) | (104) | (57) | (100) | (62) | (100) | (75) |
| Dünndarm (g) | 13,0 | 14,2 | 12,9 | 14,9 | 16,0 | 13,7 | 16,7 | 13,6 | 7,8 | 27,0 | 33,4 | 37,4 |
| ± s | 2,7 | 3,3 | 2,9 | 3,1 | 2,3 | 2,8 | 2,9 | 2,7 | 5,9 | 5,7 | 7,0 | 11,1 |
| Dünndarm (%) * | 5,2 | 7,9 | 4,8 | 7,5 | 5,0 | 8,3 | 5,0 | 7,5 | 4,1 | 6,8 | 2,9 | 4,6 |
| ± s | 0,9 | 1,8 | 1,0 | 2,0 | 0,6 | 1,4 | 0,6 | 1,4 | 0,7 | 2,3 | 0,7 | 2,8 |
| Relativ | (100) | (152) | (100) | (156) | (100) | (166) | (100) | (150) | (100) | (166) | (100) | (159) |

* Gewicht in % der Lebendmasse

Tabelle 3:Versuchsergebnisse mit nucleasebehandeltem Bakterienprotein

VB = Versuchsbeginn, VE = Versuchsende, LM = Lebendmasse

s = Streuung

| Versuch | I | | II | | III | |
|---|---|---|---|---|---|---|
| Proteinquelle | Soja | Bakterien-protein | Soja | Bakterien-protein | Soja | Bakterien-protein |
| Vitamin $D_3$ | oral | oral | oral | oral | oral | oral |
| Vit.$D_3$-Menge | 500 | IE/kg | 13000 | IE/kg | 13000 | IE/kg |
| Tierzahl | 35 | 20 | 49 | 47 | 18 | 20 |
| Vorperiode (Tg) | 4 | 4 | 4 | 4 | 4 | 4 |
| Zwischenp. (Tg) | 0 | 0 | 0 | 0 | 21 | 21 |
| Versuchsp. (Tg) | 7 | 7 | 14 | 14 | 9 | 9 |
| LM (g) bei VB | 79 | 79 | 87 | 87 | 863 | 866 |
| LM (g) bei VE | 254 | 179 | 580 | 297 | 1320 | 1196 |
| ± s | 35 | 23 | 47 | 55 | 89 | 109 |
| Relativ | (100) | (78) | (100) | (51) | (100) | (91) |
| Dünndarm (g) | 13,0 | 11,0 | 23,4 | 16,0 | 39,4 | 40,4 |
| ± s | 2,7 | 2,4 | 4,2 | 3,2 | 5,6 | 7,8 |
| Dünndarm (%) | 5,2 | 5,6 | 4,0 | 5,5 | 3,0 | 3,4 |
| ± s | 0,9 | 1,2 | 0,6 | 1,1 | 0,3 | 0,8 |
| Relativ | (100) | (109) | (100) | (138) | (100) | (113) |

Die folgenden Beispiele zeigen die Rohprotein-Veredelung.

Beispiel 1:

Methylomonas clara (ATCC 31 226) wurde in einer Nährlösung, enthaltend Methanol als einzige Kohlenstoffquelle, Ammoniak als einzige Stickstoffquelle, Phosphat, Eisen- und Magnesiumsalze sowie andere übliche Spurenelemente unter aeroben Bedingungen gezüchtet (US-PS 4 166 004). Die Konzentration der dabei erzeugten Bakterienzellmasse wurde durch Separation von 1,5 auf 15 Gew.-% erhöht.

Von dieser Paste wurden 1000 g unter langsamem Rühren auf 55°C erwärmt, der pH-Wert von ursprünglich 6,9 mit Schwefelsäure auf 5,1 gestellt und 1 mg Nuclease (Amano RP8), gelöst in 10 ml Wasser, zugefügt.

Über einen Zeitraum von 4 Stunden wurde unter Konstanthaltung von pH-Wert und Temperatur die Nucleinsäurehydrolyse durchgeführt und der Gehalt an gebildeten monomeren 5'-Nucleotiden über HPLC-Messung bestimmt. Nach 3,5 Stunden war die Reaktion praktisch beendet, d.h. der Gehalt an 5'-Nucleotiden (in Gew.-%) der eingesetzten Biomasse (150 g) entsprach nahezu quantitaitv (96 %) dem Gehalt an makromolekularer Nucleinsäure (12,6 Gew.-%) der Biomasse.

Anschließend wurde der pH-Wert mit Natronlauge auf pH 6,5 gestellt,  10 Min. bei 80°C gerührt und die so erhaltene Bioproteinmasse getrocknet.

Beispiel 2:

Es wurde wie in Beispiel 1 verfahren, jedoch 0,05 mg Phosphodiesterase (aus Crotalus durissus, Fa. Boehringer Mannheim, EC 3.1.4.1) verwendet. Die Hydrolyseausbeute an freien 5'-Nucleotiden betrug 89 %.

Beispiel 3:

Es wurde wie in Beispiel 1 verfahren, jedoch 0,15 mg Nuclease P 1 (Fa. Sigma, aus Penicillium citrinum) verwendet. Die Hydrolyseausbeute nach 3,5 Stunden betrug 97 %.

Beispiel 4:

Es wurde wie in Beispiel 1 verfahren, jedoch 10 g Weizenkeimlinge als Rohnucleasepräparation verwendet. Die Ausbeute an 5'-Nucleotiden betrug nach 3,5 Stunden 78 %.

Beispiel 5:

Es wurde verfahren wie in Beispiel 4, jedoch mit einer Rohenzyminkubationszeit von 5 Stunden. Die Ausbeute an 5'-Nucleotiden betrug 94 %.

Beispiel 6:

Die Herstellung der Biomasse erfolgte wie in Beispiel 1, jedoch wurde das Rohbioprotein nach der Separation 10 Minuten auf 85°C erhitzt, auf 55°C abgekühlt und dann mit 0,05 mg Nuclease RP8 (Amano) versetzt. Nach 2 Stunden betrug die Hydrolyseausbeute an 5'-Nucleotiden 97 %.

Beispiel 7

Aus einer kontinuierlichen Fermentation von Methylomonas clara (ATCC 31 226) gemäß US-PS 4 166 004 mit anschließender Aufkonzentrierung der Biomasse auf 30 Gew.-% und Sprühtrocknung wurden 15 kg des so erhaltenen Trockenproduktes (Restfeuchte 4,5 %, Nucleinsäuregehalt 11,8 %, bezogen auf Trockensubstanz) in 85 l Wasser unter Rühren suspendiert, der pH-Wert mit Schwefelsäure von 6,8 auf 5,4 gestellt, die Temperatur auf 55°C erhöht und 150 mg Nuclease (Sigma, EC 3.1.30.1), in 10 ml Wasser gelöst, zugesetzt.

Unter pH- (5,4) und Temperatursteuerung (55°C) wurde 3 Stunden gerührt, der pH-Wert mit Natronlauge auf 6,5 gestellt und die Suspension bei 95°C sprühgetrocknet. Die Hydrolyseausbeute an freien 5'-Nucleotiden wurde über HPLC mit 97 % bestimmt.

Beispiel 8:
Das Verfahren wurde wie in Beispiel 7 durchgeführt, jedoch wurden aus 15 kg getrocknetem Rohbioprotein gemäß dem Verfahren nach der US-PS 4 206 243, Beispiel 1, die Lipide extrahiert und die verbleibende Menge von 13 kg entfettetem Bioprotein unter den Bedingungen von Beispiel 7 mit 75 mg Nuclease (Sigma) behandelt. Die Hydrolyseausbeute an freien 5'-Nucleotiden betrug 99 %.

Beispiel 9:
Ein Kohlenwasserstoffe verwertender Stamm der Hefe Candida lipolytica (ATCC 20 383) wurde auf n-Paraffinen in Gegenwart eines wäßrigen Nährmediums und eines Sauerstoff enthaltenden Gases kultiviert (US-PS 4 206 243, Beispiel 8). Die Hefezellmasse wurde von der Nährlösung abgetrennt und getrocknet, der Nucleinsäuregehalt, bezogen auf Trockensubstanz, betrug 7,9 %.

150 g dieser Trockenmasse wurde bei 55°C in 850 ml Wasser suspendiert, der pH-Wert mit Phosphorsäure auf 5,5 gestellt und die Mischung unter Rühren und Konstanthaltung von pH-Wert und Temperatur 4 Stunden mit 3 mg Nuclease (Amano RP 8) inkubiert. Anschließend wurde die Temperatur für 10 Minuten auf 90°C erhöht, der pH-Wert mit Natronlauge auf 6,5 gestellt und getrocknet. Die Hydrolyseausbeute an 5'-Nucleotiden betrug 91 %.

0188004

PATENTANSPRÜCHE:                                    HOE 85/F 005

1. Verwendung von nucleinsäurereichen Rohproteinen, in denen durch Behandlung mit Nucleasen die hochmolekularen Nucleinsäuren gespalten wurden, zur Tierernährung.

2. Verwendung nach Anspruch 1, dadurch gekennzeichnet, daß das Rohprotein ein mikrobielles Protein ist.

3. Verwendung nach Anspruch 1 oder 2, dadurch gekennzeichnet, daß das Rohprotein ein Einzellerprotein ist.

4. Verwendung nach einem oder mehreren der vorhergehenden Ansprüche, dadurch gekennzeichnet, daß das Rohprotein ein Hefe- oder Bakterienprotein ist.

5. Verwendung nach einem oder mehreren der vorhergehenden Ansprüche, dadurch gekennzeichnet, daß das Rohprotein ein Isolat von M. clara ATCC 31226 ist.

6. Verwendung nach Anspruch 5, dadurch gekennzeichnet, daß das Isolat durch Extraktion mit einer Lösung von Ammoniak in einem niederen Alkanol, niederen Glykol oder einem Ether aus einem niederen Alkanol und einem niederen Glykol, die höchstens 30 % Wasser, bezogen auf das Gewicht des organischen Lösemittels, enthält, entfettet ist.

7. Verwendung nach einem oder mehreren der vorhergehenden Ansprüche, dadurch gekennzeichnet, daß die Nuclease 3'- oder 5'-Phosphodiesterase ist.

8. Verwendung nach einem oder mehreren der vorhergehenden Ansprüche, dadurch gekennzeichnet, daß die Spaltung bei 30 bis 60°C, einem pH-Bereich von 4,5 bis 6,5, einer
Enzymkonzentration von 0,1 bis 0,005 %, bezogen auf das
Substratgewicht, und einer Nucleinsäurekonzentration
von 0,05 bis 10 Gew.-% erfolgt.

9. Verwendung nach Anspruch 8, dadurch gekennzeichnet, daß
die Spaltung bei 45 bis 55°C, einem pH-Bereich von 5
bis 6, einer Enzymkonzentration von 0,01 bis 0,001 %,
bezogen auf das Substratgewicht, und einer Nucleinsäurekonzentration von 0,3 bis 7 Gew.-% erfolgt.

10. Verwendung nach einem oder mehreren der vorhergehenden
Ansprüche, dadurch gekennzeichnet, daß die Nucleinsäuren
bis zur Stufe der Mononucleotide gespalten sind.